# EUROPÄISCHE PATENTSCHRIFT

(11) **EP 0 739 609 B1**
(45) Veröffentlichungstag und Bekanntmachung des Hinweises auf die Patenterteilung: **10.01.2001**
(21) Anmeldenummer: 96104292.6
(22) Anmeldetag: 19.03.1996
(51) Int. Cl.: A61B 17/225

(54) **Lithotripsievorrichtung**
Lithotripsy device
Dispositif de lithotripsy

(30) Priorität: 25.03.1995 DE 19511106
(43) Veröffentlichungstag der Anmeldung: 30.10.1996
(73) Patentinhaber: Dornier MedTech Holding International GmbH, 82234 Wessling (DE)
(72) Erfinder: Gregg, Hans, 82237 Wörthsee (DE); Artmeier, Theo, 82194 Gröbenzell (DE)

(56) Entgegenhaltungen:
- EP-A- 0 372 174
- EP-A- 0 397 980
- DE-C- 4 232 683
- US-A- 4 984 575

## Beschreibung

Die Erfindung betrifft eine Lithotripsievorrichtung für die Ortung und nichtinvasive Zertrümmerung von Konkrementen im Körper eines Patienten, gemäß dem Oberbegriff des Patentanspruchs 1.

Eine derartige Lithotripsievorrichtung ist aus der EP-B-0 483 138 bekannt. Demnach ist an einer Gerätesäule ein vertikal angeordneter, ringförmiger Tragarm um eine horizontale Achse schwenkbar gelagert. An diesem Tragarm ist ein Röntgengerät befestigt, welches aus einer Röntgenquelle sowie einem dieser gegenüberliegenden Bildaufnehmer besteht, wobei im Betrieb der Röntgenzentralstrahl die horizontale Drehachse senkrecht schneidet. Die beiden genannten Komponenten des Röntgengerätes sind an dem ringförmigen Tragarm um 180° gegeneinander versetzt angebracht. Dazwischen befindet sich, ebenfalls an dem Tragarm gelagert, eine Stoßwellenquelle, welche durch radiale Verschiebung so justiert werden kann, daß ihr Therapiefokus genau im Schnittpunkt des Röntgenzentralstrahles mit der horizontalen Drehachse zu liegen kommt. Dieser Schnittpunkt bildet gleichzeitig den Mittelpunkt des ring- bzw. kreisförmigen Tragarmes. Diese Anordnung ermöglicht es, daß ein einmal in den Therapiefokus gebrachtes Konkrement auch beim Schwenken des Röntgengerätes einschließlich der ebenfalls am ringförmigen Tragarm befestigten Stoßwellenquelle um die horizontale Drehachse stets sowohl im Therapiefokus als auch in der Blickrichtung des Röntgenzentralstrahles verbleibt.

Die Patientenliege ist bei der bekannten Vorrichtung offenbar im Gesamtgerät integriert und mit ihrer Längsachse parallel zu der erwähnten Drehachse ausgerichtet. Zur Steinortung und -behandlung durch die Stoßwellenquelle muss die Liege mit dem Patienten in den ringförmigen Tragarm eingeführt werden, so dass der Stein mindestens in den Sichtbereich des Röntgengerätes gelangt. Die räumliche Lage des Steins kann dann mit Hilfe des Röntgengerätes durch zwei Aufnahmen in verschiedenen Winkelstellungen bestimmt werden. Danach wird die Liege computergesteuert so bewegt, dass das Konkrement in den Fokus der Stoßwellenquelle platziert wird.

Der geschilderte Aufbau der bekannten Vorrichtung hat somit zur Folge. dass nur die am Gerät integrierte Liege, welche für die Einführung in den ringförmigen Tragarm ausgelegt ist, benutzt werden kann. In vielen Fällen ist es aber erwünscht, den Patienten auf einer separaten Liege heranzutransportieren und ihn ohne Umlagerung auf dieser Liege einer Untersuchung und Behandlung zu unterziehen. Dies ist mit der bekannten Vorrichtung nicht möglich.

Aus der EP-A-0372 174 ist eine Vorrichtung gemäß Oberbegriff des Anspruchs 1 bekannt, bei der Stosswellenquelle und Röntgenstrahler gemeinsam in XYZ-Richtung relativ zum Patienten verfahrbar sind. Dabei sind die Stosswellenquelle und der Röntgenstrahler in einem gemeinsamen Gehäuse untergebracht.

Der Erfindung liegt daher die Aufgabe zugrunde, eine Lithotripsievorrichtung der eingangs genannten Art bereitzustellen, welche so ausgebildet ist, dass der Patient auf einer separaten Patientenliege herantransportiert werde kann, so dass unmittelbar die Steinortung mit Hilfe des Röntgengerätes sowie die Behandlung mittels der Stosswellenquelle erfolgen kann.

Diese Aufgabe wird gemäß der Erfindung durch die im kennzeichnenden Teil des Patentanspruchs 1 aufgeführten Merkmale gelöst.

Demnach ist der Tragarm nunmehr in an sich bekannter Weise in der Art eines runden oder winkeligen C-Bogens ausgebildet, wobei die horizontale Drehachse in der durch die Arme des C-Bogens gebildeten Ebene liegt. In Ortungs- bzw. Behandlungsstellung der Patientenliege ist diese horizontale Drehachse im wesentlichen senkrecht zur Längsachse der Liege orientiert. Weiterhin sind Tragarm und Stoßwellenquelle gemeinsam in drei zueinander orthogonalen Raumrichtungen bewegbar.

Durch diese Anordnung wird es möglich, eine separate, beispielsweise fahrbare Patientenliege mit dem auf ihr liegenden Patienten ohne jede Beschränkung soweit heranzufahren, daß der interessierende Körperteil des Patienten in den Sichtbereich des Röntgengerätes und damit den Fokusbereich der Stoßwellenquelle gelangt. Die besondere Orientierung der horizontalen Drehachse des Tragarmes, nämlich im wesentlichen senkrecht zur Längsachse der in Ortungs- bzw. Behandlungsstellung befindlichen Patientenliege, ermöglicht es, das Röntgengerät um diese Drehachse in zwei unterschiedliche Winkelstellungen zu verschwenken, ohne daß dadurch die an der Liege stehende behandelnde Person im Kopf- oder Beinbereich behindert würde, wie dies beispielsweise der Fall wäre, wem in der oben diskutierten, bekannten Vorrichtung der ringförmige Tragarm lediglich durch einen C-Bogen ersetzt würde. Nach Feststellung der räumlichen Koordinaten des behandelnden Konkrements mittels zweier Röntgenaufnahmen aus unterschiedlichen Winkelpositionen braucht nun, anders als bei der bekannten Vorrichtung, nicht die Liege bewegt zu werden, welche somit auch nicht über entsprechende motorische Antriebe verfügen muß, sondern der Tragarm einschließlich des daran befestigten Röntgengerätes sowie die Stoßwellenquelle können nun gemeinsam durch Bewegung in drei zueinander orthogonalen Raumrichtungen in die gewünschte Position gebracht werden, in welcher das Konkrement in den Fokus der Stoßwellenquelle zu liegen kommt, welcher weiterhin auf der durch den Zentralstrahl des Röntgengerätes gegebenen Linie verbleibt. Diese Bewegung kann sowohl unter ständiger Beobachtung eines an das Röntgengerät angeschlossenen Bildschirmes per Hand erfolgen oder auch nach Berechnung der räumlichen Koordinaten im Anschluß an die zwei Röntgenaufnahmen motor- und ggf. computergesteuert.

Eine weitere Möglichkeit der Bewegung des Röntgengerätes ist in Unteranspruch 2 angegeben, wonach der Tragarm um eine zur genannten horizontalen Drehachse senkrechte weitere Drehachse gesondert schwenkbar ist, wobei allerdings der Zentralstrahl des Röntgengerätes aus dem Fokusbereich der Stoßwellenquelle herausgeschwenkt wird. Das Röntgengerät befindet sich dann quasi in einer Parkstellung, so daß der Patient auf der Patientenliege nunmehr einer visuellen Untersuchung ohne jede Behinderung durch die Röntgenquelle unterzogen werden kann. Das herausgeschwenkte Röntgengerät kann währenddessen auch zu einer weiteren Untersuchung etwa zu urologischen Zwecken herangezogen werden.

Im folgenden wird die Erfindung in einer Ausführungsform anhand von Abbildungen näher erläutert. Es zeigen in schematischer Weise:
- Fig. 1: eine Lithotripsievorrichtung gemäß der Erfindung mit einer Patientenliege in Ortungs- bzw. Behandlungsstellung,
- Fig. 2: eine der Fig. 1 entsprechende Darstellung mit geänderter Position von Konkrement und Fokusbereich,
- Fig. 3: eine Darstellung derselben Lithotripsievorrichtung in Seitenansicht und
- Fig. 4: eine Darstellung in Draufsicht.

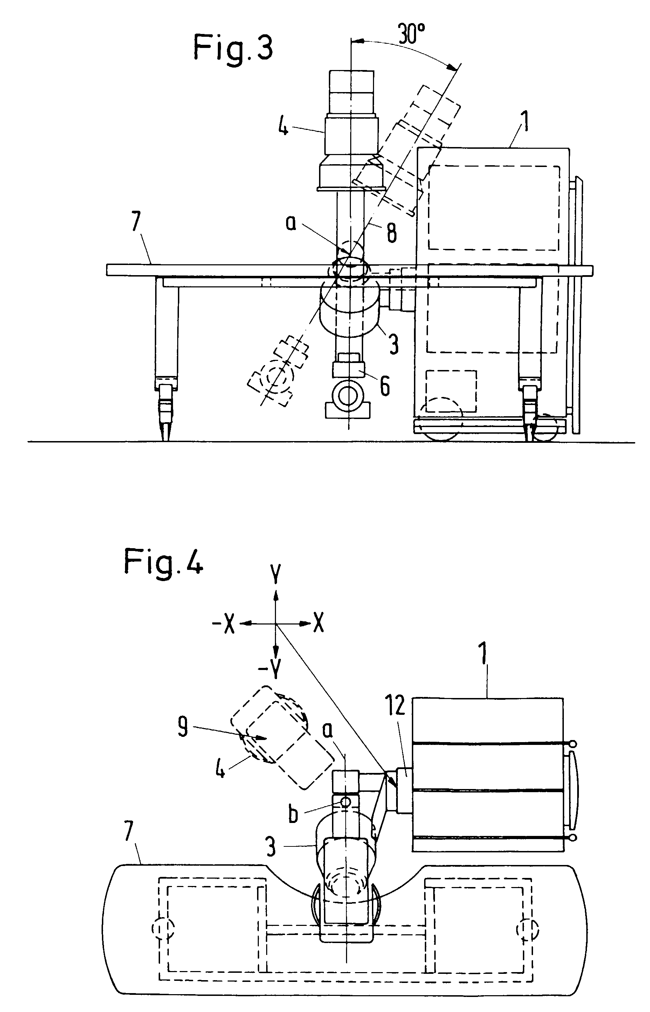

Gemäß Fig. list eine fahrbare Patientenliege 7 an eine Gerätesäule 1 herangefahren. An dieser ist ein in Form eines winkeligen C-Bogens ausgebildeter Tragarm 2 um eine horizontale Drehachse a schwenkbar gelagert. An den einander gegenüberliegenden Enden des Tragarmes 2 sind die beiden Komponenten eines Röntgengerätes, nämlich eine Röntgenquelle 4 sowie ein Bildaufnehmer 6, angebracht. Die durch den Röntgenzentralstrahl gegebene Linie ist mit 8 bezeichnet. Der Tragarm 2 ist zusätzlich um eine vertikale Drehachse b schwenkbar, so daß beispielsweise die Röntgenquelle 4 dann die in Fig 4 mit 9 bezeichnete Parkstellung einnimmt. Eine Stoßwellenquelle 3 ist nach ordnungsgemäßer Justierung so orientiert, daß ihr Fokus F auf der Linie 8 liegt.

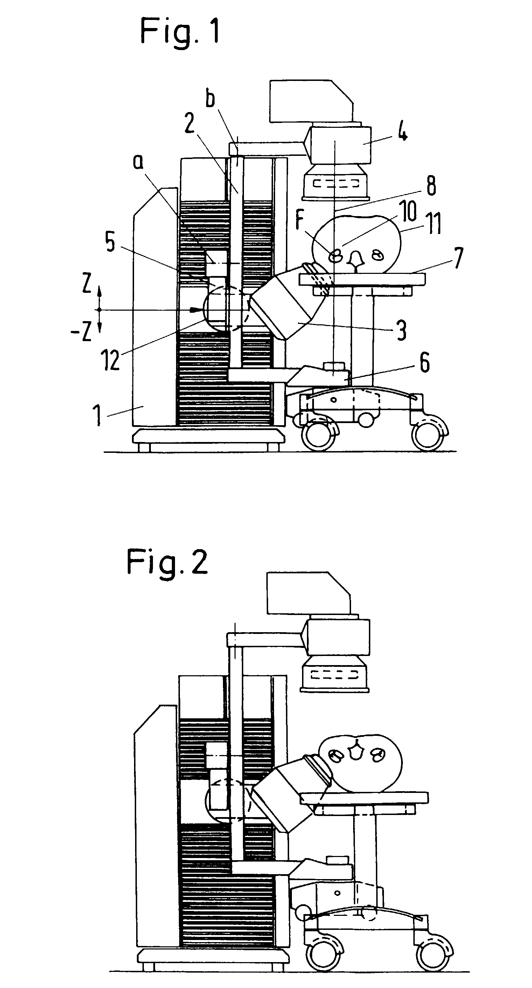

Die Stoßwellenquelle 3 sowie der Tragarm 2 können, wie in Fig. 1 sowie Fig. 4 angedeutet, gemeinsam in den drei zueinander orthogonalen Raumrichtung X, Y sowie Z bewegt werden. Dadurch wird es möglich, den Fokus F der Stoßwellenquelle 3 an den Ort des interessierenden Konkrementes 10 innerhalb des in Fig. 1 im Querschnitt dargestellten Patienten 11 zu bringen. Dies kann nach vorheriger Vermessung der räumlichen Lage dieses Konkrementes durch das Röntgengerät aus zwei unterschiedlichen Winkelstellungen, wie beispielsweise in Fig. 3 dargestellt, heraus geschehen.

Wie in Fig. 4 dargestellt, ragt aus der Gerätesäule 1 seitlich ein Tragelement 12 heraus, welches zunächst in positiver und negativer Y-Richtung verfahrbar ist. In diesem wiederum ist ein weiteres Element gelagert, auf welches in Fig. 4 der vom Achsenkreuz kommende Pfeil gerichtet ist, welches für die Bewegung in der positiven und negativen X-Richtung zuständig ist. An diesem Element ist auch mit einer entsprechenden Halterungsvorrichtung die Stoßwellenquelle 3 befestigt. Eine weitere Halterung 5 trägt gemäß Fig. 1 die horizontale Drehachse a. Insbesondere den Figuren 1 und 3 ist weiterhin zu entnehmen, daß in justierter Position der Stoßwellenquelle 3 die horizontale Drehachse a und die dem Röntgenzentralstrahl entsprechende Gerade 8 sich im Fokus F der Stoßwellenquelle 3 kreuzen. Hierdurch ist sichergestellt, daß ein einmal in den Fokus F gebrachtes Konkrement 10 auch nach Schwenken des Tragarmes 2 um die horizontale Drehachse a im Zentrum des Röntgenbildes verbleibt.

In Fig. 2 ist die Lage des Patienten insoweit verändert, als er gegenüber der in Fig. 1 dargestellten Rückenlage nunmehr die Bauchlage einnimmt. Die Stoßwellenquelle 3 muß daher in Z-Richtung bewegt werden, wie aus einem Vergleich der beiden Figuren leicht deutlich wird.

## Patentansprüche

1. Lithotripsievorrichtung für die Ortung und nichtinvasive Behandlung von Konkrementen im Körper eines auf einer Patientenliege (7) befindlichen Patienten mittels fokussierter Stoßwellen, mit einem über einen Tragarm (2) an einer Gerätesäule (1) um eine horizontale Drehachse (a) schwenkbar gelagerten Röntgengerät (4,6) , welches eine an einer Seite des Tragarmes (2) befestigte Röntgenquelle (4) und einen dieser gegenüberliegenden, an der anderen Seite des Tragarmes (2) angebrachten Bildaufnehmer (6) aufweist, sowie einer Stoßwellenquelle (3), deren Fokus (F) auf der durch den Zentralstrahl des Röntgengerätes (4,6) gegebenen Linie liegt, wobei der Tragarm (2) als runder oder winkeliger C-Bogen ausgebildet ist, die Drehachse (a) in der durch die Arme des C-Bogens gebildeten Ebene liegt und in Ortungs- bzw. Behandlungsstellung der Patientenliege im wesentlichen senkrecht zu deren Längsachse orientierbar ist und der Tragarm (2) sowie die Stoßwellenquelle (3) gemeinsam in drei zueinander orthogonalen Richtungen (XYZ) bewegbar sind, wobei die Bewegung in der horizontalen X-Y Ebene mittels eines in Y-Richtung verfahrbaren Tragelements (12) und einem im Tragelement (12) gelagerten, weiteren in X-Richtung verfahrbarem Element erfolgt,
dadurch gekennzeichnet, dass
die Stoßwellenquelle (3) mittels einer Halterungsvorrichtung und der Tragarm (2) mittels einer weiteren Halterungsvorrichtung (5) an dem in X-Richtung verfahrbarem Element befestigt sind.

2. Vorrichtung nach Anspruch 1
dadurch gekennzeichnet, dass
der Tragarm (2) zusätzlich um eine weitere, zur horizontalen Drehachse (a) senkrecht orientierte , vertikale Drehachse (b) schwenkbar ist.

## Claims

1. Lithotripsy device for location and non-invasive treatment of concrements in the body of a patient, placed on a patient table (7) by means of focussed shock waves which is equipped with an X-ray device (4,6) movable around a horizontal rotation axis (a), resting on a support arm (2) that is fixed on a device column (1) and with an X-ray source (4), placed on one side of the support arm, and an image processor unit (6), placed opposite of that source on the other side of the support arm (2), as well as with a shock wave source (3), whose focus (F) is lying on the line, designed by the central beam of the X-ray device (4,6), whereby the support arm (2) has the form of a C, round or with an angle, and the rotation axis (a) is lying on the level formed by the arms of the C. and may be oriented mostly vertical to its length (x) axis, while the patient table is in location or treatment position, and the support arm (2) as well as the shock wave source (3) are together movable in three directions, that are orthogonal to each other; whereby the motion on the horizontal x-y level is executed by means of a support element (1), movable in direction of the y axis and a further support element, fixed on this support element (12), movable in direction of the x axis,
which is characterized in that
the shock wave source (3) is fixed by means of a support and the support arm (2) by means of a further support (5) on this element, which is movable in direction of the x axis.

2. Device according to claim 1
characterized in that
additionally, the support arm (2) can be moved around a further vertical rotation axis (b), which is oriented horizontal to the horizontal rotation axis (a).

## Revendications

1. Appareil de lithotripsie pour la location et le traitement non invasives des concréments qui se trouvent dans le corps d'un patient, placé sur une couchette de patient (7), traité à l'aide des ondes de choc focalisées qui est equipé d'un appareil à rayons (4,6) qui peut etre tourné autour d'un axe de rotation horizontal (a), restant sur un support (2) qui est fixé sur la colonne d'appareil (1) et d'une source radiographique (4), placée sur l'une côtée du bras de support, et une unité processeur d'images (6), placée en face de cette source sur l'autre côtée du bras de support (2), et en plus, d'une source des ondes de choc (3), dont le foyer (F) est situé sur la ligne, dessinée par le rayon central venant de l'appareil à rayons (4,6), le bras de support (2) ayant la forme d'un C, arrondi ou avec un angle et l'axe de rotation (a) est situé sur le niveau formé par les bras du C et peut être orienté essentiellement vertical à son axe de longitude (x). Durant cela la couchette de patient se trouve en position de location ou de traitement et le bras de support (2) ensemble avec la source des ondes de choc (3) peuvent être tournés en trois directions, orthogonales l'une à l'autre, durant cela le mouvement horizontal au niveau x-y est exécuté par un élément de support (1), qui peut être tourné en direction de l'axe y, et par un autre élément de support fixé sur cet élément de support (12), qui peut être tourné en direction de l'axe x,
characterisé par le fait
que la source des ondes de choc (3) est fixée par un support, et le bras de support (2) lui-même à l'aide d'un autre support (5), sur cet élément qui peut être tourné en direction de l'axe x.

2. Appareil selon demande de brevet 1
characterisé par le fait
que, de plus, le bras de support (2) peut être tourné autour d'un autre axe de rotation vertical (b), en position horizontal à l'axe de rotation horizontal (a).
